# EUROPÄISCHE PATENTSCHRIFT

(11) **EP 1 021 198 B1**
(45) Veröffentlichungstag und Bekanntmachung des Hinweises auf die Patenterteilung: **27.02.2002**
(21) Anmeldenummer: 98958807.4
(22) Anmeldetag: 29.09.1998
(51) Int. Cl.: A61K 35/78, A61K 31/335, A61P 1/16

(54) **FLAVONOLIGNAN-ZUBEREITUNGEN, INSBESONDERE SILYMARIN-ZUBEREITUNGEN**
FLAVONOLIGNAN PREPARATIONS, ESPECIALLY SILYMARIN PREPARATIONS
PREPARATIONS DE FLAVONOLIGNAN, NOTAMMENT PREPARATIONS A BASE DE SILYMARINE

(30) Priorität: 08.10.1997 DE 19744459
(43) Veröffentlichungstag der Anmeldung: 26.07.2000
(73) Patentinhaber: Willmar Schwabe GmbH & Co., 76209 Karlsruhe (DE)
(72) Erfinder: HERRMANN, Joachim, D-76229 Karlsruhe (DE); OSCHMANN, Rainer, D-76829 Landau (DE); STUMPF, Heinz, D-76228 Karlsruhe (DE)
(74) Vertreter: Rudolph, Ulrike, Dr.
(86) Internationale Anmeldenummer: DE9802943
(87) Internationale Veröffentlichungsnummer: WO9918985

(56) Entgegenhaltungen:
- EP-A- 0 278 809
- EP-A- 0 300 282
- DE-A- 2 017 789

## Beschreibung

Die Erfindung betrifft die Verwendung von silymarinhaltigen Flavonolignan-Zubereitungen mit im Vergleich zu reinen Flavonoiden bzw. reinem Silymarin verbessertem Auflöseverhalten, d.h. insbesondere verbesserter Freisetzung und infolgedessen verbesserter Resorbierbarkeit und Bioverfügbarkeit im tierischen oder menschlichen Körper, zur Herstellung von im tierischen oder menschlichen Körper zur Auflösung und Resorption kommenden Arzneimittel zur Therapie und Prophylaxe von Lebererkrankungen und ein Verfahren zur Herstellung dieser Arzneimittel.

Unter den Arzneimitteln zur Therapie und Prophylaxe von Lebererkrankungen spielen solche auf der Basis von Mariendistelextrakten (Carduus-Extrakten) eine immer bedeutendere Rolle. Wirksamer Bestandteil der Mariendistelextrakt-Zubereitung ist Silymarin, bestehend aus den vier Flavonoid-Isomeren (Flavonolignan-Isomere) Silibinin, Isosilibinin, Silicristin und Silidianin. Da Silymarin bzw. Flavonoide in Wasser nur sehr wenig löslich sind, (die Löslichkeit von reinem Silymarin in pH 6,9 Puffer liegt bei ca. 0,08 mg/ml), besteht bei all diesen Arzneimitteln das Problem, daß ihre Resorbierbarkeit und damit ihre Bioverfügbarkeit im menschlichen oder tierischen Körper häufig nur unbefriedigend gering ist. Außerdem neigen die Flavonoid-Isomere dazu, mit vielen der üblichen galenischen Hilfsstoffen schwer lösliche Agglomerate zu bilden. Um die Wasserlöslichkeit zu verbessern, wurde bereits versucht, die Flavonoid-Isomere des Silymarin mit speziellen chemischen Agenzien zu Derivaten (Addukte, Komplexverbindungen, Ester Einschlußverbindungen) umzusetzen, die eine bessere Wasserlöslichkeit und eine höhere Freisetzungsrate aufweisen. Ein wesentlicher Nachteil dieser Derivate besteht jedoch darin, daß es sich dabei im Prinzip um neue Wirkstoffe handelt, und daß die an die Flavonoid-Isomere gekoppelten Agenzien oftmals unerwünschte physiologische Nebenreaktionen hervorrufen können und/oder auch die Wirksamkeit der Flavonoid-Isomere beeinträchtigenkönnen. Diese Nachteile überwiegen vielfach gegenüber dem Vorteil der verbesserten Wasserlöslichkeit. Grundsätzlich ist eine biopharmazeutische Beeinflussung der Resorbierbarkeit und Bioverfügbarkeit über die Optimierung der Arzneiform in jedem Fall vorzuziehen.

Ein anderer Weg zur Verbesserung von Lösungsverhalten, Freisetzungsrate und Bioverfügbarkeit ist in der Druckschrift EP 0 722 719 beschrieben. Demgemäß werden die Flavonoid-Isomere zusammen mit pharmazeutisch annehmbaren Trägerstoffen und Netzmitteln in wässrig-alkoholischem Medium aufgelöst und diese Lösung zur Bildung eines Copräzipitats konzentriert, filtriert, unter Vakuum getrocknet und pulverisiert. Die auf diese Art und Weise erhaltenen Silymarin-Copräzipitate weisen eine gegenüber dem reinen Wirkstoff erheblich höhere Freisetzungsrate (von 95-100%) und Bioverfügbarkeit auf, sie werden im menschlichen oder tierischen Körper sehr gut resorbiert und entfalten die gewünschte und erwartete physiologische Wirkung. Dieses Herstellungsverfahren ist jedoch'technisch sehr aufwendig und verursacht relativ hohe Kosten, vor allem dadurch bedingt, daß (1) mehrere Hilfsstoffe benötigt werden, daß (2) Lösungsmittel zum Einsatz kommen, die später wieder entfernt werden müssen, daß (3) eine Vermahlung notwendig ist, und daß (4) der Wirkstoff einer thermischen Belastung ausgesetzt ist. Mit der Erhitzung der Ausgangslösung bis zum Siedepunkt geht außerdem der Nachteil einher, daß bei einer derartigen thermischen Belastung die Wahrscheinlichkeit von chemischen Veränderungen des Wirkstoffes erhöht ist.
Ein ganz ähnlicher Lösungsweg ist auch schon in der DE 27 19 581 A1 beschrieben worden. Bei dem dort aufgezeigten Verfahren wird der Wirkstoff Polyhydoxyphenylchromanon aus Silybum marianum zusammen mit Polyvinylpyrrolidon in einem wässrigorganischen Lösungsmittel aufgelöst und diese Lösung lyophilisiert, wobei ein oberflächenaktives Mittel zugesetzt werden kann.

Ein Verfahren zur Herstellung einer Pflanzenextrakt-Zubereitung, bei dem auf eine Trocknung und Vermahlung ausdrücklich verzichtet wird, ist zwar bereits aus der EP 0496 705 bekannt. Diese Druckschrift schlägt die Herstellung einer Masse aus einem flüssigen Teil- oder Vollextrakt aus frischen und/oder getrockneten Pflanzen(-teilen) und wenigstens einem Trägermaterial, wie z.B. Polyethylenglycol oder Polysorbat, im Masse-Verhältnis zwischen 20:1 und 200:1 vor. In der Liste der für den Pflanzenextrakt in Frage kommenden Pflanzen ist Silybum marianum bzw. Carduus bzw. Mariendistel jedoch nicht aufgeführt, und auch das Problem der schlechten Auflösung, Freisetzungsrate und Bioverfögbarkeit von Flavonoiden ist in dieser Druckschrift nirgends tangiert. Überdies enthält der dort beschriebene Pflanzen-Roh- oder-Primärextrakt grundsätzlich immer noch signifikante Restmengen des bzw. der zu seiner Herstellung eingesetzten Lösungsmittel(s) und auch nach der abschließenden Einengung enthält das zu verkapselnde Endprodukt noch Restlösungsmittelmengen. Schließlich enthält keine der dort beschriebenen konfektionsfertigen Zubereitungen den Pflanzenextrakt in vollständig oder auch nur weitgehend gelöster Form.

Aufgabe der vorliegenden Erfindung ist deshalb die Bereitstellung einer Silymarin-Zubereitung, in der die Flavonoid-Isomere chemisch weitgehend naturbelassen, d.h. insbesondere nicht an andere chemische Substanzen gebunden vorliegen, und die eine hohe Silymarin-Freisetzungsrate, d.h. ein sehr gutes Silymarin-Auflöseverhalten und damit die Voraussetzung für eine gute Resorption und eine hohe Bioverfügbarkeit der Flavonoid-Isomere im menschlichen oder tierischen Körper aufweisen. Die für die Zubereitung notwendigen Hilfsstoffe sollen physiologisch völlig unbedenklich und zahlenmäßig eng begrenzt sein, d.h. es sollen möglichst wenig verschiedene Hilfsstoffe eingesetzt werden.

Eine Lösung dieser Aufgabe besteht in der Verwendung einer silymarinhaltigen Flavonolignanzubereitung, die ausschließlich oder nahezu ausschließlich aus einer klaren, nahezu oder vollständig homogenen, flüssigen Mischung aus Mariendistel-Trockenextrakt (Carduus-Extrakt) mit hohem Silymarin-Gehalt in Polyethylenglykol (PEG) bestehen,― ohne Verwendung von zusätzlichen Trägerstoffen, zur Herstellung eines im tierischen oder menschlichen Körper zur Auflösung und Resorption kommenden Arzneimittels zur Therapie und Prophylaxe von Lebererkrankungen. Es wurde nämlich überraschenderweise gefunden, daß PEG als alleiniges Lösungsmittel für den Mariendistel-Trockenextrakt auf technisch einfachste Art und Weise zu einer Mariendistel-Extrakt-Zubreitung führt, die ein praktisch gleich gutes Auflöseverhalten aufweist wie die in der EP 0 722 719 A1 beschriebenen Copräzipitate und die in der Publikation von H.-U. Schulz et al., "Untersuchungen zum Freisetzungsverhalten und zur Bioäquivalenz von Silymarin-Präparaten", Arznein.-Forsch./Drug Res. 45(I), Nr. 1, 1995, getesteten und bewerteten Arzneimittel.

Die für die erfindungsgemäße Verwendung vorgeschlagene Zubereitung hat die Vorteile, daß sie sich in Wasser sehr gut verteilt und daß sie den Wirkstoff Silymarin mit vergleichsweise sehr hoher Rate freisetzt. Die ermittelten Werte für die in-vitro Freisetzungsrate sind mit denen von Copräzipitat-Zubereitungen mindestens gleichwertig (vgl. Tabelle 2).
Im Unterschied zu dieser bekannten Zubereitung ist die für die erfindungsgemäße Verwendung vorgesehene Zubereitung jedoch mit einem wesentlich geringeren technischen Aufwand und erheblich kostengünstiger herstellbar. Es entfällt insbesondere die Vermahlung und solche Herstellungsschritte, bei denen besondere Lösungsmittel eingesetzt werden, die nachfolgend wieder entfernt und als Abfall entsorgt oder aufgearbeitet werden müssen.

Bei dem eingesetzten Polyethylenglykol handelt es sich vorzugsweise um PEG 200, PEG 300, PEG 400 oder PEG 600.

In einer bevorzugten Variante der für die erfindungsgemäße Verwendung vorgesehene Zubereitung liegt das Verhältnis von Mariendistel-Trockenextrakt zu Polyethylenglykol (PEG) im Bereich zwischen 1 : 10 und 1 : 1,5, vorzugsweise zwischen 1 : 5 und 1 : 2. Diese Zubereitung zeigt ein außerordentlich gutes Auflöseverhalten, sowohl im Vergleich zu den in der EP 0 722 719 genannten Werten als auch im Vergleich zu den in der Publikation von Schulz, H.-U. et al.,: "Untersuchungen zum Freisetzungsverhalten und zur Bioäquivalenz von Silymarin-Präparaten", Arzneim.-Forsch./Drug Res. 45(I), Nr. 1 (1995) veröffentlichen Werten diverser Silymarin-Präparate. Eine Zubereitung mit diesbezüglich hervorragen guten Eigenschaften enthält das Mariendistel-Trockenextrakt (Carduus-Extrakt) in Polyethylenglykol 400 im Masse-Verhältnis 1 : 3.

Die für die erfindungsgemäße Verwendung vorgeschlagene Zubereitung kann Zusätze von pharmazeutisch allgemein akzeptierten Hilfsstoffen und Cosolventien enthalten. Die Zugabe von Cosolventien ist insbesondere dann von Vorteil, wenn die Zubereitung für eine Konfektionierung in Weichgelatinekapseln vorgesehen ist. Als Cosolvens kann Propylenglycol, Glycerol wasserfrei oder Glycerol 85% verwendet werden. Bevorzugte Cosolventien sind Glycerol oder Propylenglykol.
Darüber hinaus können weitere Zusätze, z.B. Tenside, insbesondere Polysorbat 80 (Tween 80®), hinzugefügt werden.

Zur Herstellung der für die erfindungsgemäße Verwendung vorgesehenen Zubereitungen wird ein Verfahren vorgeschlagen, das durch die Anzahl und Reihenfolge der folgenden Verfahrensschritte charakterisiert ist:
(a) Erwärmen von flüssigem Polyethylenlglykol, vorzugsweise auf ca 50°C,
(b) Einrühren eines auf übliche Weise erhaltenen Mariendistel-Trockenextraktes in diese erwärmte Lösung und intensives Homogenisieren,
(c) Zugabe von Cosolventien und/oder Hilfsstoff(en)
(d) Homogenisieren unter intensivem Verrühren bis zum Erhalt einer nahezu oder vollständig homogenen flüssigen Mischung
wobei die Verfahrensschritte (a), (b) und (d) zwingend sind, während der Verfahrensschritt (c) nur bei Bedarf, d.h. wenn die Zugabe von Cosolventien und/oder Hilfsstoffen erforderlich ist, durchgeführt wird bzw. entfällt, wenn die Zubereitungen frei von Cosolventien und Hilfsstoffen sind.

Die erfindungsgemäße Zubereitung ist insbesondere zur Abfüllung in Gelatine-Kapseln, vorzugsweise Weichgelatinekapseln, hergestellt aus normaler Gelatine oder succinylierter Gelatine, geeignet. Zu diesem Zweck wird vorgeschlagen, die Zubereitung nach dem vorstehend beschriebenen Verfahren unter Zugabe von Cosolventien und Hilfsstoffen herzustellen.

Die Erfindung wird im folgenden anhand von Herstellungsbeispielen und in-vitro-Testergebnissen näher erläutert.

### Beispiel 1: Herstellung einer Mariendistel-Extrakt-Polyethylenglykol-Zubereitung

Aus Mariendistelfrüchten (Cardui mariae fructus) wird nach einem herkömmlichen Verfahren ein fester Extrakt mit einem Silymaringehalt von ca. 80 % (photometrische Gehaltsbestimmung) hergestellt. Dieser Extrakt wird in Polyethylenglykol 400, das zu diesem Zweck auf etwa 50 °C erwärmt wurde, eingerührt. Diese Mischung wird so lange intensiv homogenisiert, bis eine homogene flüssige Mischung vorliegt, d.h. erfahrungsgemäß etwa 5 Min. lang.

### Beispiel 2: Herstellung einer Mariendistel-Extrakt-Polyethylenglykol-Zubereitung für die Abfüllung in Weichgelatinekapseln

Aus Mariendistelfrüchten (Cardui mariae fructus) wird nach herkömmlichen Verfahren ein Trockenextrakt mit einem Silymaringehalt von ca 80% (photometrische Gehaltsbestimmung) hergestellt. Dieser Extrakt wird in Polyethylenglykol 400, das zu diesem Zweck auf etwa 50 °C erwärmt wurde, eingerührt. Diese Mischung wird so lange intensiv homogenisiert, bis eine homogene flüssige Mischung vorliegt. Zu dieser Mischung wird wasserfreies Glycerol und ggf. weitere Zusätze wie z.B. Polysorbat 80 hinzugefügt. Anschließend wird wieder homogenisiert (ca. 1 Min.).

Die so erhaltene flüssige Mischung kann unter Rühren direkt in Weichgelatine-Kapseln abgefüllt werden.

### Beispiel 3: Untersuchung der Silymarin-Freisetzungsrate von Mariendistel-Extrakt-Polyethylenglykol-Zubereitungen

Die Wirkstofffreisetzung der gemäß Beispiel 1 und Beispiel 2 hergestellten Silymarin-Polyethylenglykol-Zubereitungen wurden in einer Freisetzungsapparatur (Standard Bedingungen, Paddle-Modell, gemäß DAB 10, 900 ml Phosphatpuffer pH 7,5 nach DAB10) untersucht. Aufgrund der geringen Löslichkeit des Wirkstoffs wurde ¼ der üblichen Einzeldosis, entsprechend ca. 35 mg Silymarin verwendet. Die quantitative Bestimmung der in Lösung gegangenen Silymarin Menge erfolgte photometrisch (Photometer der Fa. Perkin Elmer, Typ Lambda 14) bei 288 nm (Berechnung der Konzentration durch Vergleich mit entsprechend hergestellten Vergleichslösungen). Die Ergebnisse sind in der nachfolgenden Tabelle 1 dargestellt.

| Silymarin- Polyethylenglykol-Zubereitungen | Freigesetzter Anteil in % der eingesetzten Silymarin-Menge nach 60 Minuten (photometrisch bestimmt bei 288 nm) |
|---|---|
| gemäß Beispiel 1 | 98,3 |
| gemäß Beispiel 2 | 83,2 |

Die schnelle und möglichst vollständige Auflösung bzw. Freisetzung gewährleistet eine gute Resorption und hohe Bioverfügbarkeit des Wirkstoffs.

**Tabelle 2:**

| | |
|---|---|
| Freisetzung von Silymarin aus Mariendistel-Extrakt-Zubereitungen (Silymarin-Gehalt ca. 80 %m/m) in 900 ml Phosphat-Puffer pH 7,4 R nach DAB10, photometrische Bestimmung. Gesamtmasse an Extrakt 43,9 mg/Freisetzungsgefäß, entsprechend ¼ der üblichen Einzeldosis. | |

| Zubereitung | Freigesetzter Silyrnarinanteil (%) nach 60 Minuten |
|---|---|
| Erfindungsgemäße flüssiger Mischung aus Mariendistel-Extrakt und PEG 400/Polysorbat 80 | 98,3 |
| Vermahlenes Copräzipitat, hergestellt analog EP 0722719A1; Teilchengröße 99 % < 40 µm | 85,0 |
| Fertigarzneimittel, enthaltend Mariendistel-Extrakt | 84,0 |

## Patentansprüche

1. Verwendung einer silymarinhaltigen Flavonolignan-Zubereitung, ausschließlich oder nahezu ausschließlich bestehend aus einer klaren, nahezu oder vollständig homogenen, flüssigen Mischung aus Mariendistel-Trockenextrakt in Polyethylenglycol (PEG), zur Herstellung eines im tierischen oder menschlichen Körper zur Auflösung und Resorption kommenden Arzneimittels zur Therapie und Prophylaxe von Lebererkrankungen.

2. Verwendung nach Anspruch 1, **dadurch gekennzeichnet**
**daß** das Polyethylenglycol (PEG) PEG 200, PEG 300, PEG 400 oder PEG 600 ist.

3. Verwendung nach Anspruch 1 oder 2, **dadurch gekennzeichnet,**
**daß** das Masse-Verhältnis Mariendistel-Trockenextrakt zu Polyethylenglycol (PEG) im Bereich zwischen 1 : 10 und 1 : 1,5, vorzugsweise zwischen 1: 5 und 1 : 2, liegt.

4. Verwendung nach einem der Ansprüche 1 bis 3, **dadurch gekennzeichnet,**
**daß** die flüssige Mischung aus Mariendistel-Trockenextrakt und PEG im Masse-Verhältnis 1 : 3 besteht.

5. Verwendung nach einem der Ansprüche 1 bis 4, **dadurch gekennzeichnet,**
**daß** Zusätze von pharmazeutisch akzeptierten Cosolventien und/oder Hilfsstoffen vorgesehen sind.

6. Verwendung nach Anspruch 5, **dadurch gekennzeichnet,**
**daß** die Cosolventien Glycerol wasserfrei oder Propylenglykol sind.

7. Verwendung nach einem der Ansprüche 3 bis 6, **dadurch gekennzeichnet,**
**daß** der/die Hilfsstoff(e) Tenside, insbesondere Polysorbat 80 (Tween 80® ), ist/sind.

8. Verfahren zur Herstellung eines Arzneimittels, das zur Therapie und Prophylaxe von Lebererkrankungen geeignet ist und das ausschließlich oder nahezu ausschließlich aus einer klaren, nahezu oder vollständig homogenen, flüssigen Mischung aus Mariendistel-Trockenextrakt in Polyethylenglycol (PEG) besteht, **gekennzeichnet durch** die Anzahl und Reihenfolge der Verfahrensschritte:
(a) Erwärmen von flüssigem Polyethylenglycol, vorzugsweise auf ca 50°C,
(b) Einrühren eines Mariendistel-Trockenextrakts in diese erwärmte Lösung und intensives Homogenisieren
(c) Zugeben von Cosolventien und/oder Hilfsstoff(en)
(d) Homogenisieren der Mischung bis zum Erhalt einer nahezu oder vollständig homogenen flüssigen Mischung
wobei Verfahrensschritt (c) entfällt, wenn die Zubereitung frei von Cosolventien und Hilfsstoffen ist.

9. Verfahren nach Anspruch 8, **dadurch gekennzeichnet, daß** man die Mischung aus Mariendistel-Trockenextrakt in Polyethylenglycol (PEG) unter Zugabe von Cosolventien und Hilfsstoffen herstellt und in Gelatine-Kapseln, vorzugsweise Weichgelatinekapseln, hergestellt aus normaler Gelatine oder succinylierter Gelatine, abfüllt.

## Claims

1. A application of a silymarin-containing flavonolignan preparation, consisting exclusively or nearly exclusively of a clear, nearly or completely homogeneous, fluid mixture of Our-Lady's-thistle dry extract in polyethylene glycol (PEG), to manufacture a pharmaceutical that is dissolved and resorbed in the animal or human body for treating and preventing liver diseases.

2. A application according to claim 1, **characterized in that** the polyethylene glycol (PEG is PEG 200, PEG 300, PEG 400 or PEG 600.

3. A application according to claim 1 or 2, **characterized in that** the mass ratio of Our-Lady'-thistle dry extract to polyethylene glycol (PEG) ranges between 1 : 10 and 1 : 1.5, preferably between 1 : 5 and 1 : 2.

4. A application according to one of claims 1 to 3, **characterized in that** the fluid mixture of Our-Lady's-thistle dry extract and PEG has a mass ration of 1 : 3.

5. A application according to one of claims 1 to 4, **characterized in that** additives of pharmaceutically acceptable cosolvents and/or inactive ingredients are provided.

6. A application according to claim 5, **characterized in that** the cosolvents are anhydrous glycerol or propylene glycol.

7. A application according to one of claims 3 to 6, **characterized in that** the inactive ingredient(s) is/are surfactants, in particular Polysorbate 80 (Tween 80®).

8. A procedure for manufacturing a pharmaceutical that is suitable for treating and preventing liver diseases, and that consists exclusively or nearly exclusively of a clear, nearly or completely homogeneous, fluid mixture of Our-Lady's-thistle dry extract in polyethylene glycol (PEG), **characterized by** the number and sequence of procedural steps:
(a) heating of fluid polyethylene glycol, preferably to approx. 50 °C;
(b) blending in an Our-Lady's-thistle dry extract into this heated solution and homogenizing intensively;
(c) adding cosolvents and/or inactive ingredient(s);
(d) homogenizing the mixture until obtaining a nearly or completely homogeneous, fluid mixture,
wherein procedural step (c) is omitted if the preparation is free of cosolvents and inactive ingredients.

9. A procedure according to claim 8, **characterized in that** the mixture of Our-Lady's-thistle in polyethylene glycol (PEGT) is manufacture with the addition of cosolvents and inactive ingredients, and filled into gelatin capsules, preferably soft gelatin capsules made out of normal gelatins or succinylated gelatins.

## Revendications

1. Utilisation d'une préparation de flavonolignane contenant de la silymarine, consistant exclusivement ou presque exclusivement en un mélange liquide dilué, presque ou totalement homogène, d'extrait sec de chardon Marie dans du glycol de polyéthylène (GPE) pour la fabrication d'un médicament pouvant se dissoudre ou se résorber dans le corps animal ou humain pour la thérapie et la prophylaxie de maladies du foie.

2. Utilisation selon la revendication 1, **caractérisée en ce que** le glycol de polyéthylène (GPE) est du GPE 200, GPE 300, GPE 400 ou GPE 600.

3. Utilisation selon la revendication 1 ou 2, **caractérisée en ce que** le rapport de masse de l'extrait sec de chardon Marie par rapport au glycol de polyéthylène ( GPE) se situe dans la fourchette entre 1 : 10 et 1 : 1,5, de préférence entre 1 : 5 et 1 : 2.

4. Utilisation selon une des revendications 1 à 3, **caractérisée en ce que** le mélange liquide d'extrait sec de chardon Marie et de GPE a un rapport de masse de 1 : 3.

5. Utilisation selon une des revendications 1 à 4, **caractérisée en ce que** des additifs de co-solvants et/ou matières auxiliaires acceptés en pharmacie sont prévus.

6. Utilisation selon la revendication 5, **caractérisée en ce que** les co-solvants sont du glycérol sans eau ou du glycol de propylène.

7. Utilisation selon une des revendications 3 à 6, **caractérisée en ce que** la(les) matière(s) auxiliaire(s) est(sont) des surfactants, en particulier du polysorbate 80 (Tween 80 ®).

8. Procédé pour la fabrication d'un médicament qui est adapté pour la thérapie et la prophylaxie de maladies du foie et qui consiste exclusivement ou presque exclusivement en un mélange liquide dilué, presque ou complètement homogène, d'extrait sec de chardon Marie dans du glycol de polyéthylène (GPE), **caractérisé par** le nombre et la succession des étapes de processus :
(a) Chauffe de glycol de polyéthylène liquide, de préférence à environ 50 °C,
(b) Mélange d'un extrait sec de chardon Marie dans cette solution chauffée et homogénéisation intensive
(c) Ajout de co-solvants et/ou matière(s) auxiliaire(s)
(d) Homogénéisation du mélange jusqu'à obtention d'un mélange liquide presque ou totalement homogène
l'étape de processus (c) étant éliminée lorsque la préparation est exempte de co-solvants et matières auxiliaires.

9. Procédé selon la revendication 8, **caractérisé en ce que** l'on fabrique le mélange d'extrait sec de chardon Marie dans du glycol de polyéthylène (GPE) en y ajoutant des co-solvants et des matières auxiliaires et qu'on en remplit des gélules en gélatine, de préférence des gélules en gélatine molle, fabriquées à partir de gélatine normale ou de synthèse.
